# FASCICULE DE BREVET EUROPEEN

(11) **EP 2 324 870 B1**
(45) Date de publication et mention de la délivrance du brevet: **01.06.2016**
(21) Numéro de dépôt: 10016027.4
(22) Date de dépôt: 15.03.2004
(51) Int. Cl.: A61M 1/34, A61M 1/16, A61L 2/00

(54) **DISPOSITIF DE TRAITEMENT DE SANG AVEC EXTRACTION SÉLECTIVE DE SOLUTÉS**
VORRICHTUNG ZUR BLUTBEHANDLUNG MIT SELEKTIVER ENTFERNUNG GELÖSTER SUBSTANZEN
BLOOD TREATMENT DEVICE WITH SELECTIVE SOLUTE EXTRACTION

(30) Priorité: 17.03.2003 FR 0303257
(43) Date de publication de la demande: 25.05.2011
(62) Demande divisionnaire de: 04720673.5
(73) Titulaire: Gambro Lundia AB, 220 10 Lund (SE)
(72) Inventeur: Chevallet, Jacques, 69360 Sérézin du Rhône (FR); Mercier, Guy, 69500 Bron (FR); Rada, Hiram, 69008 Lyon (FR); Monchi, Mehran, 77190 Dammarie Les Lys (FR)
(74) Mandataire: Ponzellini, Gianmarco

(56) Documents cités:
- EP-A- 0 832 656
- WO-A-95/04560
- WO-A-96/28198
- DE-A1- 4 102 693
- DE-A1- 19 854 338
- US-A- 4 243 532
- US-A- 5 108 612

## Description

### Domaine de l'invention :

L'objet de la présente demande est la filtration de sang afin de séparer et extraire sélectivement des molécules de taille choisie par le biais de systèmes extracorporels pour la séparation de substances.

De tels systèmes sont utilisés pour le traitement de sang comportant des solutés de masse moléculaire différente. De telles substances sont à titre d'exemple l'urée d'une masse moléculaire de 60 daltons, le phosphate (96-97 daltons), la créatinine (113 daltons), la vitamine B12 (1355 daltons), l'inuline (5200 daltons), la béta 2-microglobuline (12000 daltons), l'albumine (58000 daltons).

On appellera par la suite molécules de petite taille les molécules dont la masse moléculaire est inférieure à environ 2000 daltons, molécules de masse moyenne les molécules dont la masse moléculaire est comprise entre 2000 et 50000 daltons et molécules de grande masse les molécules dont la masse moléculaire est supérieure à 50000 daltons (les protéines par exemple).

### Etat de la technique :

De tels systèmes sont souvent des systèmes à membrane extracorporelle pour la séparation de solutés de masse inférieure à l'albumine appliqués à des traitements d'insuffisance rénale.

Des progrès ont toujours été recherché notamment pour améliorer la clairance, pour diminuer la durée de traitement et pour rendre de tels systèmes plus simples et moins chers. On rappelle que la clairance d'un soluté est le taux de soluté dans le sang nettoyé pour un volume donné.

Dans le domaine de la dialyse, les premières membranes utilisées étaient hautement perméables à des petits solutés ayant une taille jusqu'à 200 dalton. La clairance des petits solutés dépend de la perméabilité et la capacité de diffusion de la membrane utilisée.
Le manque de perméabilité des premières membranes pour certains solutés à taille moléculaire moyenne dans la gamme des vitamines B₁₂ (1355 daltons) a été tenu responsable de l'apparition de neuropathies multiples urémiques.

Concernant l'amélioration de la clairance de moyennes molécules. une première solution était d'ajouter au flux diffusif à travers la membrane un flux convectif en utilisant des membranes à flux élevé ayant un point de coupure (« cut off » en anglais) de taille moléculaire de 40000 dalton. On rappelle que le point de cut off d'une membrane est défini par la taille moléculaire à laquelle seulement 10% des solutés passent à travers la membrane.
Mais les problèmes rencontrés lors de la mise en oeuvre de cette solution sont la difficulté de contrôler le taux d'ultrafiltration obtenu par le flux convectif, et la perte élevée de composants utiles du plasma tels que des hormones, des vitamines et des acides aminés.

Une deuxième solution concernant l'amélioration de la clairance de moyennes molécules fut alors l'hémofiltration, c'est-à-dire un procédé purement convectif d'élimination de solutés par la membrane. Mais un tel procédé extrait beaucoup de liquide et nécessite en compensation une dilution de liquide stérile en pré-dilution et / ou en post-dilution et l'utilisation d'une membrane hautement perméable aux solutés de taille moléculaire atteignant 40 000 dalton. Seulement, en mode purement convectif la clairance dépend du mode de dilution (pré ou post-dilution), du débit du sang et du débit de l'infusat. Avec une hémofiltration conventionnelle, la clairance des petites molécules est inférieure à celle obtenue en mode hémodialyse. Cette clairance en mode hémofiltration pourrait atteindre celle de l'hémodialyse si l'on augmente le débit de l'infusat, le débit sanguin et la surface de membrane. Mais tout ceci n'est pas pratique, augmente le coût du traitement et provoque la perte d'acides aminés et d'hormones. De plus, le débit sanguin est limité, notamment pour les patients ayant un accès sanguin faible.

Concernant la clairance des petites molécules, quand il a été constaté cette clairance en mode hémafiltration était limitée, les deux phénomènes d'hémofiltration et d'hémodialyse ont été combinés. Cette technique simultanée pour une même membrane est connue sous le nom d'hémodiafiltration. Mais les problèmes constatés sont la difficulté du contrôle précis du flux d'hémofiltration, la perte élevée d'hormones et d'acides aminés, la complexité du système, les grandes quantités de liquide stérile et de dialysat nécessaires et par conséquent le coût d'un tel traitement.

Ainsi, l'utilisation d'un seul filtre travaillant en modes opératoires différents ne résolvait toujours pas notamment les problèmes de pertes de molécules d'une certaine taille et de coût du traitement.

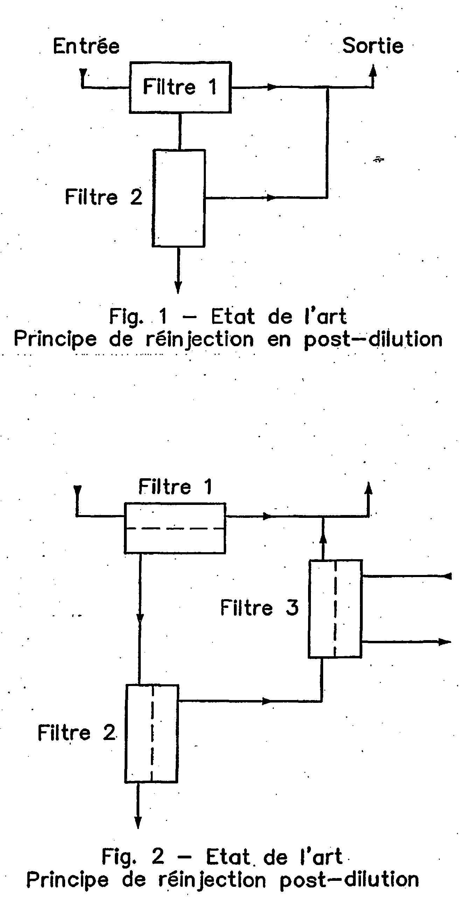

Une proposition a alors été faite par les docteurs J.C. Kingswood et F.D.Thompson. Elle avait pour objet une hémofiltration continue sans liquide de ré-injection : le traitement de l'ultrafiltrat était assuré par une deuxième membrane fonctionnant également en ultrafiltration spontanée. La figure 1 représente le dispositif de dialyse issu de cette proposition.

Il s'agit de traiter un premier ultrafiltrat issu d'une première membrane à fibres creuses par le passage à travers une seconde membrane à fibres creuses en mode ultrafiltration. Une première ultrafiltration est effectuée à travers une première membrane à flux élevé et non perméable aux molécules de masse moléculaire supérieure à 10000 dalton. La taille des orifices à travers la seconde membrane est plus petite que celle de la première.

Comme indiqué sur la figure 1, en sortie de la première membrane, le liquide non filtré contenant principalement des molécules de grande masse est amené en ré-injection au patient. Le premier ultrafiltrat contenant des molécules de petite et moyenne masse est filtré à travers la seconde membrane. Le liquide non filtré par la seconde membrane contenant principalement des molécules de masse moyenne est recueilli dans une poche de déchet. Le deuxième ultrafiltrat contenant principalement des molécules de petite masse est réinjecté en post-dilution sur la ligne veineuse du patient.

Ceci permet de ne pas consommer trop de liquide stérile en post-injection, et de réinjecter au patient un liquide contenant peu de particules de taille moyenne.
Mais une perte élevée de nutriments, d'acides aminés, de glucose et de vitamines a été constatée ; et la clairance des petits ions comme le potassium n'est pas bonne.

Aussi, un autre dispositif a été réalisé en dialyse. Il a été considéré que les molécules urémiques à éliminer avaient une masse moléculaire inférieure à 200 daltons et une masse moléculaire comprise entre 10000 et 40000 daltons.
Cette considération fut à l'origine de la réalisation d'un dispositif comportant trois filtres, représenté en figure 2.
Un premier filtre possède un point de cut off d'environ 40000 daltons. Le sang passe à travers ce premier filtre et produit en sortie un premier filtrat contenant petites et moyennes molécules, c'est-à-dire des molécules de masse moléculaire inférieure à 40000 daltons. Les solutés de masse comprise entre 10000 et 40000 daltons sont ensuite éliminés par ultrafiltration à travers le filtre 2 ayant un point de cut off inférieur à 10000 daltons. Puis le deuxième filtrat est traité en hémofiltration avec une membrane ayant un point de coupure d'environ 200 daltons. Ainsi, le filtrat purifié contenant les solutés entre 200 et 10000 daltons est retourné en post infusion vers le patient qui reçoit aussi les molécules de masse supérieure à 40000 daltons.

Mais la clairance de tous les solutés dépend du taux d'ultrafiltration dans le filtre 1, qui ne peut pas être supérieure à 30% du flux sanguin, ce qui est une valeur faible en comparaison avec l'hémodialyse conventionnelle. Aussi le coût est élevé.

Enfin, le brevet US 6,193,681 décrit un appareil pour traiter la septicémie dans le sang représenté en figure 3. Le sang passe par un dispositif d'irradiation à utraviolet puis à travers un hémoconcentrateur avant d'être ré-injecté dans le patient. Un circuit secondaire est branché à une deuxième sortie de l'hémoconcentrateur dont le fluide sortant passe à travers un filtre, puis un module de membrane et une source de dilution avant d'être injecté en amont de l'hémoconcentrateur.

Il existe par ailleurs un problème équivalent pour la plasmaphérèse. La plasmaphérèse d'échange thérapeutique est pratiquée sur un patient dont le plasma contient une ou plusieurs substances nocives ou toxiques.
L'élimination des solutés du plasma s'effectue selon le même principe que l'élimination des solutés du sang, une différence étant la plus grande masse moléculaire des solutés à extraire du plasma.

Ainsi, des problèmes récurrents ont été rencontrés lors de la réalisation des dispositifs de l'art intérieur
La consommation élevée du liquide de perfusion,
La perte élevée de nutriments, d'acides aminés, de glucose et de vitamines,
La clairance faible des solutés,
Le coût du dispositif comportant plusieurs filtres et pompes.

Le problème posé par la demande est d'éliminer sélectivement des molécules par gamme(s) de masse moléculaire avec une bonne clairance et de consommer très peu de liquide stérile. Par exemple, pour des patients en état de septicémie, on souhaite éliminer beaucoup de molécules de taille moyenne tout en gardant une élimination correcte de molécules de petite masse. On rappelle que la septicémie est caractérisée par des décharges importantes et répétées de germes pathogènes figurés à partir d'un foyer primitif.

Un problème auxiliaire serait l'adaptation optimale d'un tel système à une thérapie de longue durée pratiquée en soins intensifs sans risque d'obturation des filtres. L'adaptation s'effectuerait par le choix des modes de fonctionnement des différents filtres, de l'utilisation et du placement opportun de moyens de réglage de débit, des débits commandés, et l'architecture hydraulique des lignes.

US5108612 shows a hemofilter: filtered water is treated by an absorber and by a dialysis unit and then re-injected in pre-dilution to the hemofilter. WO96/28198 shows a plasmafilter: filtered fluid is treated by a reactor and then re-injected in pre-dilution to the plasmafilter. US4243532 shows a plasmafilter and an absorber with return of filtered plasma in pre-dilution to the plasmafiltes

### Exposé de l'invention :

Pour atteindre le problème posé on prévoit, conformément à l'invention, un dispositif de traitement extracorporel de sang selon la revendication 1. D'autres caractéristiques de l'invention apparaîtront à la lecture de la description qui suit.

### Brève description des dessins

On se reportera aux dessins annexés sur lesquels :
la figure 1 représente l'état de l'art concernant l'utilisation de deux filtres avec points de coupure différents et avec une ré-injection en post-dilution;
la figure 2 représente l'état de l'art concernant l'utilisation de trois filtres avec points de coupure différents et avec une ré-injection en post-dilution;
la figure 3 représente l'état de l'art du brevet US6,193,681;
les figures 4 à 10 sont des représentations schématiques du dispositif de traitement de liquide physiologique selon des variantes de réalisation; seulement les figures 9 et 10 concernent l'invention revendiquée.
les figures 11 et 12 représentent les résultats estimés en terme de clairance en fonction de la taille moléculaire des solutés pour deux configurations de dispositif selon l'invention.

### Exposé détaillé de l'invention :

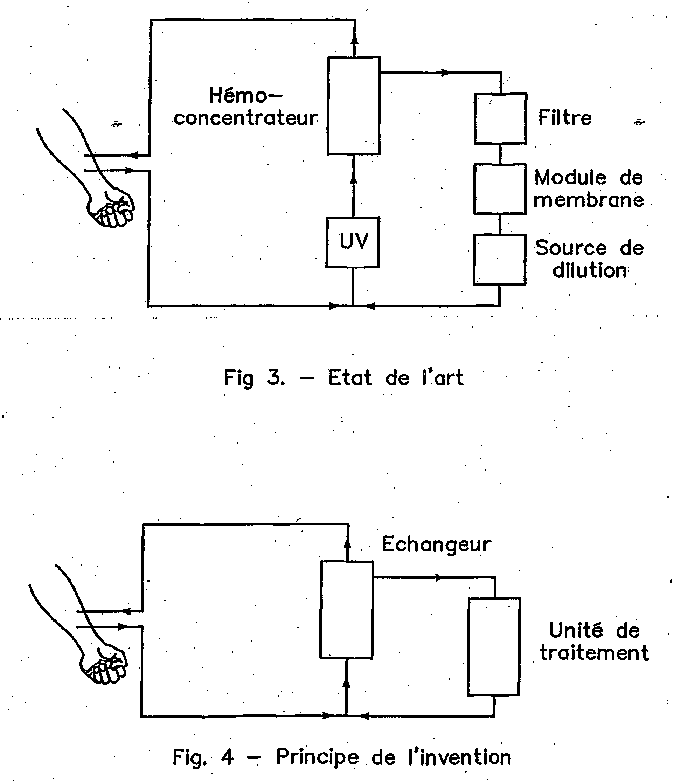

La figure 4 représente de façon schématisée le passage du sang dans une ligne d'entrée, son entrée dans un échangeur et sa sortie de l'échangeur vers une ligne de sortie, ainsi que le traitement du premier filtrat par une unité de traitement et l'injection du liquide en sortie de l'unité de traitement en pré-dilution sur la ligne artérielle. On peut voir ce concept comme une « cascade » de filtration avec injection du filtrat final en pré-dilution du circuit : un premier filtrat est une deuxième fois filtré, et le deuxième filtrat est injecté en entrée du premier filtre, ou en « pré-dilution ».

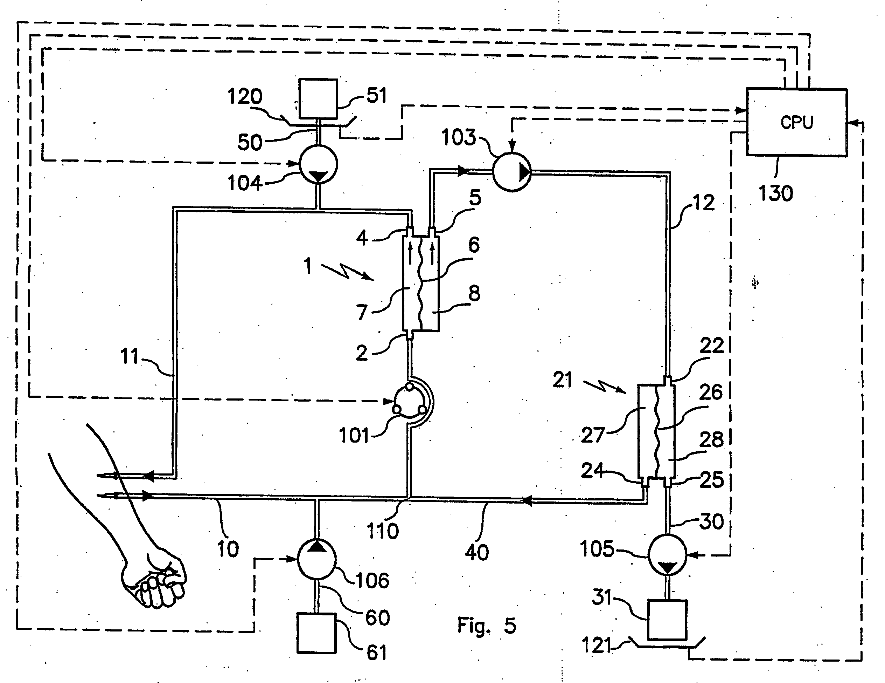

La figure 5 représente le dispositif de traitement extracorporel de sang comprenant un échangeur 1 comportant une première entrée 2 pour le sang à traiter, une première sortie 4 de fluide et une deuxième sortie 5 de fluide, une ligne d'entrée 10 du sang à traiter ou ligne artérielle raccordée à la première entrée 2 de l'échangeur 1, une ligne de sortie du sang ou ligne veineuse 11 raccordée à la première sortie 4 de l'échangeur 1. Une unité de traitement 21 comporte une première entrée 22 de fluide et une première sortie 24 de fluide, la deuxième sortie 25 de l'échangeur 1 est en communication de fluide avec la première entrée 22 de l'unité de traitement 21, et la première sortie 24 de l'unité de traitement 21 est en communication de fluide avec la ligne d'entrée 10.
La communication de fluide entre la première entrée 22 de l'unité de traitement 21 et la deuxième sortie 5 de l'échangeur 1 est effectuée par une canalisation 12.

L'échangeur 1 peut comporter une membrane semi-perméable 6 le divisant en une première chambre 7 et une deuxième chambre 8. La première entrée 2 de l'échangeur est en communication de fluide avec la première chambre 7 de l'échangeur et la première sortie 4 de l'échangeur est en communication de fluide avec la première chambre 7 de l'échangeur et la deuxième sortie 5 de l'échangeur est en communication de fluide avec la deuxième chambre 8 de l'échangeur.

La ligne d'entrée 10 du sang dite « ligne artérielle » raccordée à la première entrée 2 de l'échangeur 1, la ligne de sortie 11 du sang dite « ligne veineuse » raccordée à la première sortie 4 de l'échangeur et la première chambre 7 de l'échangeur font partie d'un circuit extracorporel de traitement de sang.

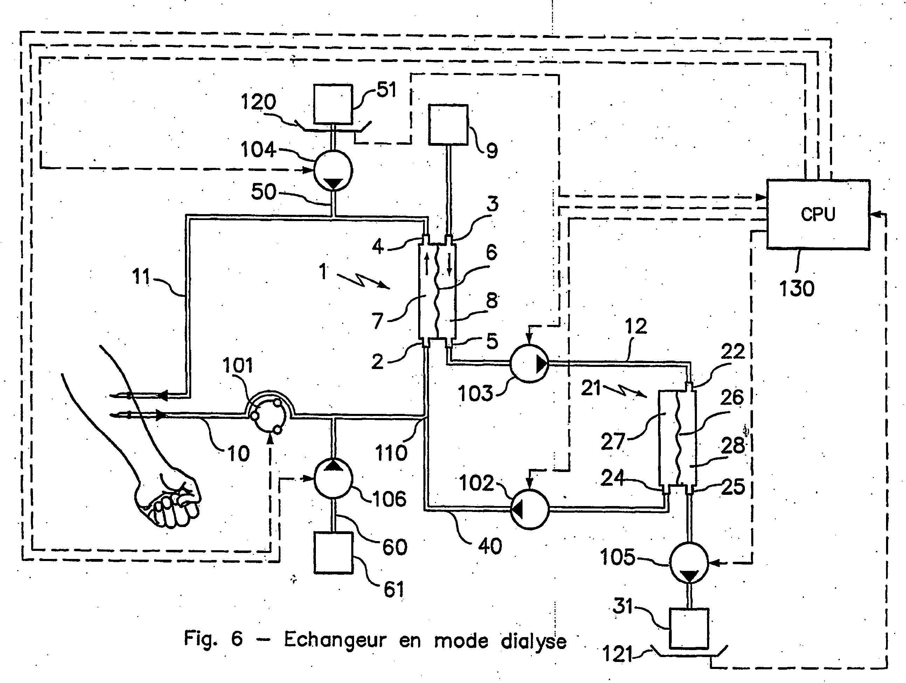

Selon figure 6, l'échangeur 1 peut comporter une deuxième entrée 3 en communication de fluide avec la deuxième chambre 8 et en communication de fluide avec une première source de liquide de dialyse 9. Dans ce mode de fonctionnement, le sang et le liquide de dialyse circulent en sens inverse dans chacune des deux chambres.

La figure 5 représente l'unité de traitement 21 avec une membrane semi-perméable 26 la divisant en une première -chambre 27 et une deuxième chambre 28.

Aussi, l'unité de traitement 21 peut comporter une deuxième sortie de fluide 25.

Aussi, la première sortie 24 de l'unité de traitement 21 est en communication de fluide avec la première chambre 27 de l'unité de traitement 21 et la deuxième sortie 25 de l'unité de traitement 21 est en communication de fluide avec la deuxième chambre 28 de l'unité de traitement 21.

De manière alternative, la première entrée 22 de l'unité de traitement 21 peut être en communication de fluide soit avec la deuxième chambre 28 de l'unité de traitement 21 soit avec la première chambre 27 de l'unité de traitement 21.

La deuxième sortie 25 de l'unité de traitement 21 est en communication de fluide avec une première ligne de décharge 30 de liquide usé, ladite première ligne de décharge 30 pouvant relier la deuxième sortie 25 de l'unité de traitement 21 à un égout ou à un premier réservoir de liquide usé 31.

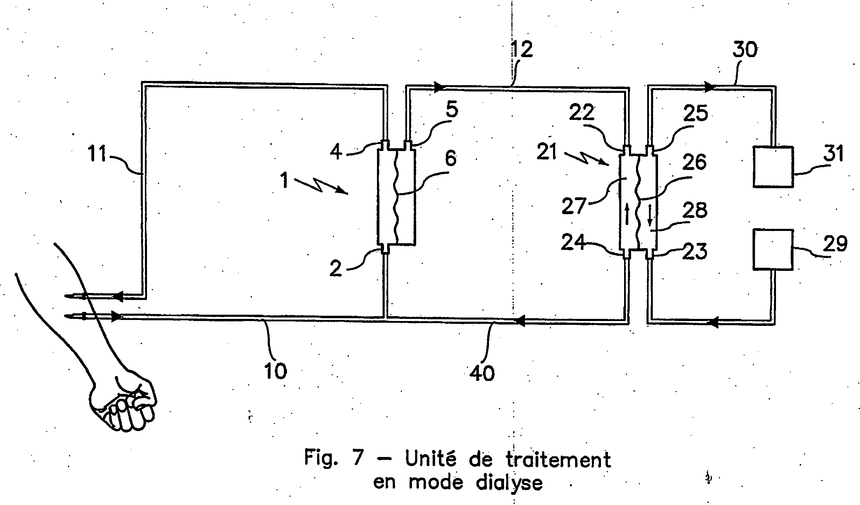

L'unité de traitement 21 peut également comporter une deuxième entrée 23, la deuxième entrée 23 étant en communication de fluide avec la deuxième chambre 28 et avec une deuxième source de liquide de dialyse 29. Dans ce mode de fonctionnement de l'unité de traitement représenté en figure 7, le liquide de dialyse circule en contresens par rapport au liquide physiologique arrivant par la première entrée 22.

L'échangeur 1 et l'unité de traitement 21 ont des caractéristiques différentes. En effet, la membrane 6 de l'échangeur 1 peut être une membrane à flux élevé et la membrane 26 de l'unité de traitement 21 peut être une membrane à flux faible.
Une membrane à flux faible a une perméabilité à l'eau faible. Le coefficient d'ultrafiltration est approximativement entre 2 et 10mL/h,mmHg,m². Une membrane à flux élevé a une perméabilité à l'eau plus élevée. Le coefficient d'ultrafiltration est approximativement entre 20 et. 50mL /h, mmHg, m².

L'échangeur ou l'unité de traitement peut comporter une membrane à fibres creuses (on parle également de filtre capillaire) ou une membrane à fibres planes, c'est-à-dire à plaques.

Aussi, la perméabilité aux molécules de la membrane 6 de l'échangeur 1 est supérieure à la perméabilité aux molécules de la membrane 26 de l'unité de traitement 21 au moins au-dessus d'une certaine masse moléculaire.
Plus particulièrement, on peut définir un rapport ou une différence entre les points de coupure de la première membrane et de la deuxième membrane. Ainsi, on peut considérer que le rapport du point de coupure de la première membrane sur le point de coupure de la deuxième membrane est inférieur ou égal à 3. D'une autre manière, on peut considérer que la différence de point de coupure entre la première membrane et la deuxième membrane est comprise entre 20000 et 30000 daltons. On peut envisager que le point de coupure de la première membrane soit inférieur ou égal à 40000 daltons, que le point de coupure de la deuxième membrane soit inférieur ou égal à 10000 daltons. Dans un mode utilisé le point de coupure de la première membrane est approximativement égal à 40 000 daltons et le point de coupure de la deuxième membrane est approximativement égal à 10 000 daltons.

Afin de ré-infuser de l'eau au patient en traitement, on peut brancher sur la ligne de sortie 11 une ligne de post-dilution 50 reliée à une première source de liquide stérile 51 et/ou sur la ligne d'entrée 10 une ligne de pré-dilution 60 reliée à une deuxième source de liquide stérile 61.

Une canalisation 40 met en communication de fluide la première sortie 24 de l'unité de traitement 21 et la première entrée 2 de l'échangeur 1.

La ligne de pré-dilution 60 peut être branchée directement sur ladite canalisation 40 ou directement sur la ligne d'entrée 10.

Les différentes sources de liquide stérile 51, 61 peuvent être des poches de liquide stérile et/ou peuvent être obtenues par une préparation en ligne de liquide stérile à partir de l'eau du réseau hydrique.

### Selon

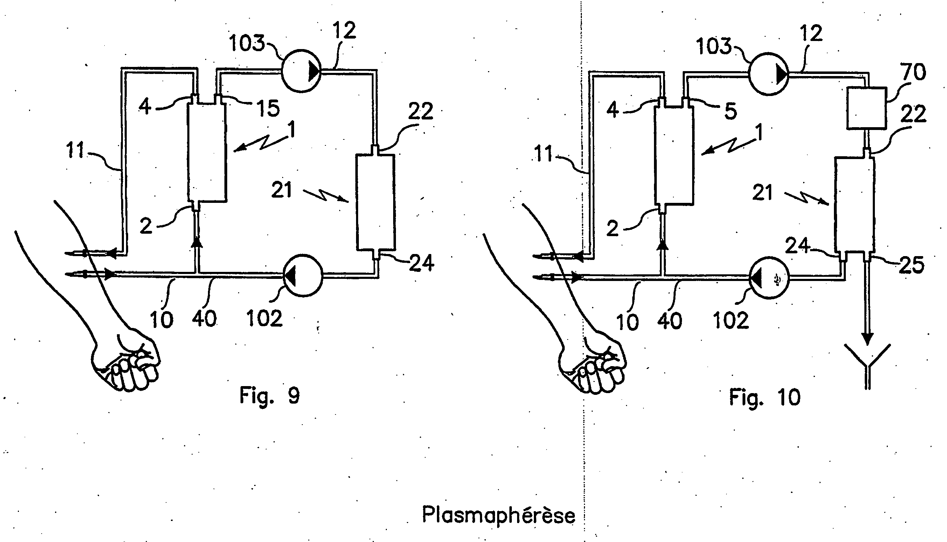

Selon la présente invention, comme représenté en figures 9 et 10, l'échangeur est un plasma-filtre. Le plasma-filtre a un point de coupure compris entre un million et cinq million daltons.

Là encore, l'échangeur ou l'unité de traitement peut comporter une membrane à fibres creuses (on parle également de filtre capillaire) ou une membrane à fibres planes, c'est-à-dire à plaques.

Aussi, l'unité de traitement 21 comprend une unité capable de fixer au moins une substance donnée. Elle peut être une cartouche d'adsorption, un réacteur.

L'unité de traitement peut comporter une membrane semi-perméable 26 la divisant en une première chambre 27 comportant une première sortie 24 et une deuxième chambre 28 comportant une première entrée 22 et une deuxième sortie 25. La deuxième sortie est reliée à une ligne de décharge. L'unité de traitement peut avoir un point de coupure inférieur ou égal à 250000 daltons.

Le point de coupure pourra être inférieur ou égal à 200000 daltons.

L'unité de traitement peut avoir un point de coupure tel que la membrane laisse passer 100% des molécules d'albumine à 58000 daltons.

La figure 10 représente un dispositif comportant des moyens de réaction sur au moins certaines molécules 70. Ces moyens sont branchés sur la conduite 12 entre la deuxième sortie 5 de l'échangeur 1 à la première entrée 22 de l'unité de traitement 21. Ces moyens pour réagir sur au moins certaines molécules 70 peuvent être un réacteur, un adsorbeur, un dispositif de rayonnement, par exemple un dispositif d'électrophérèse, de réaction enzymatique, d'irradiation à ultraviolet. le plasma-filtre peut alors avoir des pores d'un micron. L'unité de traitement peut avoir un point de coupure inférieur ou égal à 90000 daltons laissant passer les protéines vers le sang du patient.

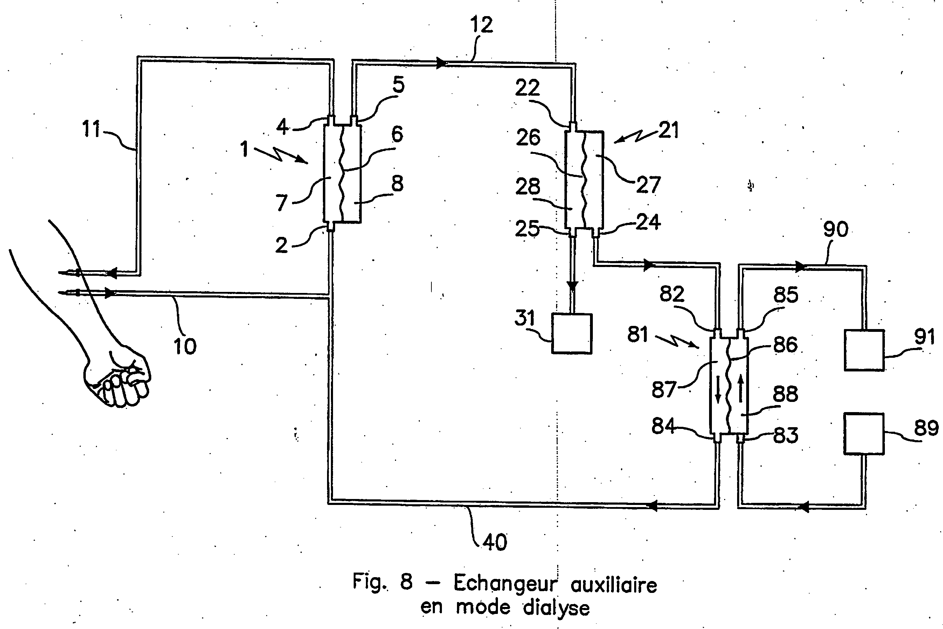

Un autre aspect est de rajouter un troisième moyen de filtration pour effectuer une élimination supplémentaire de gamme de masse moléculaire, représenté en figure 8. Le dispositif peut comporter au moins un échangeur auxiliaire 81 ayant une membrane 86 le séparant en une première chambre 87 en communication de fluide avec une première entrée 82 et une première sortie 84 et en une deuxième chambre 88 en communication de fluide avec au moins une deuxième sortie 85. Le point de coupure d'un tel échangeur auxiliaire serait inférieur aux points de coupure des deux autres membranes (6, 26).
La première entrée 82 de l'échangeur auxiliaire 81 est en communication de fluide avec la deuxième sortie 24 de l'unité de traitement 21 et une des deux sorties 84, 85 de l'échangeur auxiliaire 81 est en communication de fluide avec la première entrée 2 de l'échangeur 1.

Une deuxième ligne de décharge 90 de liquide usé relie l'autre sortie 84, 85 de l'échangeur auxiliaire 81 à un égout, l'égout pouvant être un deuxième réservoir de liquide usé 91.

La figure 8 représente l'échangeur auxiliaire fonctionnant en mode dialyse : l'échangeur auxiliaire 81 comporte une deuxième entrée 83 en communication de fluide avec la deuxième chambre 88 de l'échangeur auxiliaire 81 et en communication de fluide avec une troisième source de liquide de dialyse 89, la première sortie 84 de l'échangeur auxiliaire 81 étant en communication de fluide avec la première entrée 82 de l'échangeur 1, la deuxième sortie 85 de l'échangeur auxiliaire 81 étant en communication de fluide avec un égout 91 par une deuxième ligne de décharge de liquide usé 90.

Le choix des trois membranes sera effectué très précisément en fonction du patient et du traitement à adopter concernant la masse des molécules que l'on souhaite éliminer ou garder. La première membrane 6 permet de travailler sur les molécules de grande masse moléculaire (de façon préférée en mode hémofiltration), la deuxième membrane 26 permet de travailler sur les molécules de moyenne masse moléculaire (de façon préférée en mode hémofiltration), et la troisième membrane 86 permet de travailler sur les molécules de petite masse moléculaire donc de façon préférée en mode dialyse. Ceci n'empêche pas l'échangeur auxiliaire 81 de fonctionner en mode ultrafiltration également. Le choix du fonctionnement permet de personnaliser le traitement et d'accéder à un fonctionnement optimal des membranes sans trop d'obturation.

Concernant le réglage des différents débits de fluide, il est prévu des premiers moyens de réglage de débit 101 de liquide actifs sur la ligne d'entrée 10 reliée à la première entrée 2 de l'échangeur 1.
Alternativement, les premiers moyens de réglage de débit 101 peuvent être exactement entre la première entrée 2 de l'échangeur 1 et le point de branchement 110 reliant la ligne d'entrée à la canalisation ou en amont du point de branchement 110 reliant la ligne d'entrée 10 à la canalisation 40.
Dans la première alternative, la dépression de la canalisation 40 nécessite une pression positive plus faible sur la conduite 12 pour atteindre la pression trans-membranaire (TMP) de la membrane 26 souhaitée.
Aussi, dans la première alternative, il n'est pas nécessaire d'avoir une pompe sur la canalisation 40 : une seule pompe 101 suffit pour la canalisation 40 et la ligne artérielle 11.

Dans la deuxième alternative, des deuxièmes moyens de réglage de débit 102 de liquide actifs sont sur la deuxième canalisation 40 reliant la première sortie 24 de l'unité de traitement 21 à la première entrée 2 de l'échangeur 1.

Il est également prévu des troisièmes moyens de réglage de débit 103 de liquide actifs sur la première conduite 12 reliant la deuxième sortie 5 de l'échangeur 1 à une des entrées 22, 23 de l'unité de traitement 21.
Aussi des quatrièmes moyens de réglage de débit 104 de liquide actifs peuvent être branchés sur la ligne de post-dilution 50.
Des cinquièmes moyens de réglage de débit de liquide 105 actifs peuvent être branchés sur la ligne de décharge 30 de liquide usé reliant la deuxième sortie 25 de l'unité de traitement 21 à un égout 31.
Dans la configuration ayant au moins les trois moyens de réglage de débit 101 sur la ligne d'entrée, 102 sur la deuxième canalisation 40 et 105 sur la ligne de décharge 30, il faudra veiller tout particulièrement aux différents débits imposés et compatibles.
Des sixièmes moyens de réglage de débit de liquide 106 actifs peuvent être branchés sur la ligne de pré-dilution 60.
Ces moyens de réglage de débit 101, 102, 103, 104 et 105 peuvent être des pompes et/ou des vannes. En particulier les moyens de réglage de débit sur la ligne de décharge 30, ou sur la ligne 50 de post-dilution ou la ligne 60 de pré-dilution seront des vannes.

Dans un mode, la première source 51 de liquide stérile pour la post-dilution est une poche de liquide stérile et le premier réservoir 31 de liquide usé relié à la ligne de décharge en sortie de l'unité de traitement est une poche de liquide usé. Le dispositif comporte une premiêre balance 120 pour mesurer le poids de la poche de liquide stérile 51 et une deuxième balance 121 pour mesurer le poids de la poche de liquide usé 31. Alternativement, une unique balance 120, 121 peut mesurer le poids total de la poche de liquide stérile 51 et de la poche de liquide usé 31.

Dès lors, une unité de calcul et de commande 130 va recevoir les signaux émis par au moins une balance 120, 121 et contrôler les moyens de réglage de débit de liquide 101, 102, 103, 104, 105.

L'unité de calcul et de commande calcule périodiquement le débit réel ou un paramètre fonction du débit réel par exemple à partir du poids et de l'intervalle de temps entre chaque deux mesures. Elle comparera le débit réel mesuré au débit souhaité et sera en mesure de contrôler un ou plusieurs moyens de réglage de débit de liquide (101, 102, 103, 104, 105).

Ainsi, les quantités de liquide stérile et de liquide usé, ou leur différence peuvent être connues et contrôlées pendant le traitement. Connaissant lesdits poids, l'unité de commande et de contrôle pourra obtenir une quantité souhaitée de solution liquide stérile et de liquide usé. L'équilibre hydrique pourra être bien commandé.

Le dispositif décrit précédemment est applicable à la plasmaphérèse.

Un procédé de traitement extracorporel de sang pour la mise en oeuvre sur un dispositif de traitement extracorporel de sang comportant un échangeur 1 sur lequel sont branchées une ligne d'entrée 10 du sang et une ligne de sortie 11 du sang, et une unité de traitement 21, comprend les étapes suivantes :
envoyer le sang sur la ligne d'entrée 10 reliée à la première entrée de l' échangeur 1,
effectuer une première filtration du sang via l'échangeur 1 en produisant un premier filtrat sortant par la deuxième sortie de l'échangeur,
effectuer au moins une deuxième filtration du premier filtrat via l'unité de traitement 21 en produisant un deuxième filtrat,
renvoyer le deuxième filtrat sortant de la première sortie de l'unité de traitement sur la ligne d'entrée 10 pour effectuer une prê-dilution du sang à traiter,
envoyer le sang à travers la première sortie de l'échangeur vers la ligne de sortie 11.

Particulièrement, le procédé aura une deuxième filtration effectuée à travers une membrane semi-perméable 26 dans une unité de traitement 21 divisée en une première chambre 27 et une deuxième chambre 28 produisant en sortie d'une part le deuxième filtrat et envoyant d'autre part en sortie le liquide non filtré vers une ligne d'égout 30.

Une autre caractéristique du procédé sera que la première filtration est effectuée à travers une membrane semi-perméable 6 divisant de l'échangeur 1 en une première chambre 7 et une deuxième chambre 8.

Une autre caractéristique du procédé sera que la membrane 26 de l'unité de traitement filtre des molécules de masse moléculaire inférieure à la masse moléculaire des molécules filtrées de la membrane 16 de l'échangeur.

Selon une autre caractéristique, le procédé comprenant l'étape de perfuser un liquide stérile dans la ligne de sortie 11 du sang de l'échangeur.

Selon une autre caractéristique, le procédé comprend l'étape de perfuser un liquide stérile dans la ligne d'entrée 10 du sang de l'échangeur.

Selon une autre caractéristique, le procédé emploie une membrane 16 de l'échangeur ayant un point de coupure inférieur à 40000 daltons.

Selon une autre caractéristique, le procédé emploie une membrane 16 de l'unité de traitement ayant un point de coupure inférieur à 10000 daltons.

Selon une caractéristique de l'invention, le traitement effectué est une plasmaphérèse et l'unité de traitement fixe au moins une certaine substance donnée.

Selon une autre caractéristique de l'invention, la membrane 16 de l'échangeur a un point de coupure compris entre un million et cinq millions daltons.

Selon une autre caractéristique de l'invention, la membrane 16 de l'unité de traitement a un point de coupure inférieur à 250000 daltons.

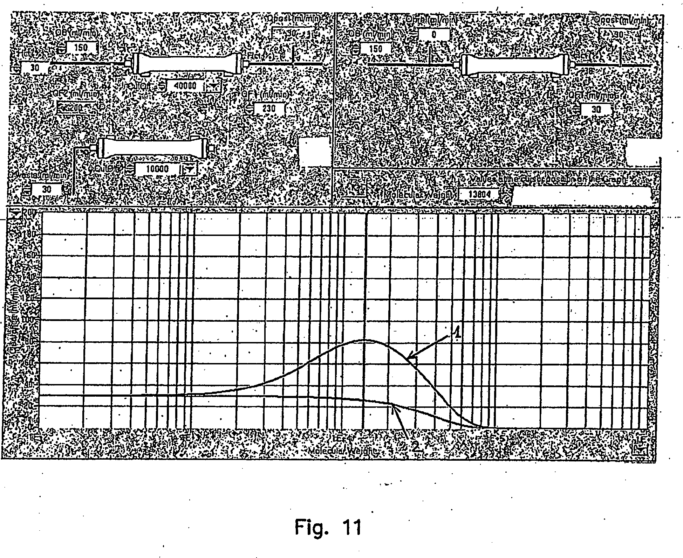

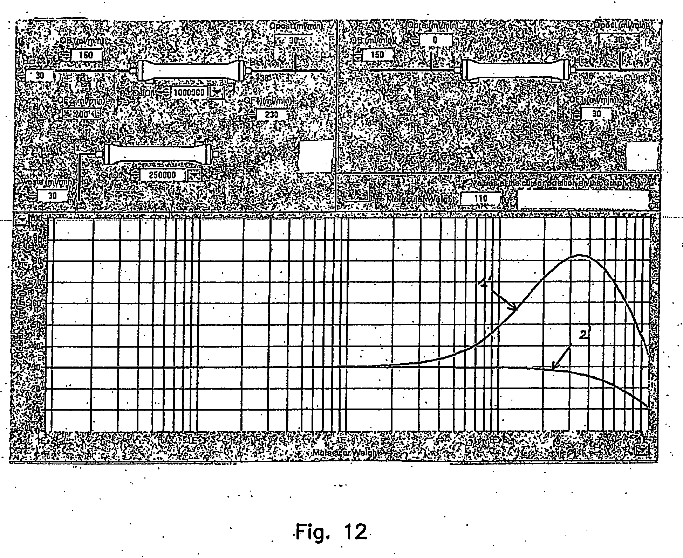

Des simulations ont été faites concernant des filtres à points de coupure différents. Les figures 11 et 12 représentent les résultats estimés en terme de clairance en fonction de la masse moléculaire des solutés pour deux configurations de dispositif selon l'invention. La figure 11 représente une première configuration ayant un échangeur de point de coupure égal à 40000 daltons, et une unité de traitement ayant un échangeur de point de coupure égal à 10000 daltons. La clairance (courbe 1) pour des molécules autour de 11000 daltons est très bonne alors que la clairance des petites molécules est gardée constante par rapport à un dispositif de fonctionnement ayant un unique filtre (courbe 2).

La figure 12 représente une deuxième configuration pour la plasmaphérèse ayant un échangeur de point de coupure égal à 1 000 000 daltons, et une unité de traitement ayant un échangeur de point de coupure égal à 250 000 daltons. La clairance (courbe 1') pour des molécules autour de 300 000 daltons est très bonne alors que la clairance des moyennes molécules est gardée constante par rapport à un dispositif de fonctionnement ayant un unique filtre (courbe 2').

### Les solutions décrites ci-dessus comportent

Les solutions décrites ci-dessus comportent de nombreux avantages:
- multiplier par trois ou quatre, par rapport à un traitement standard de longue durée, l'épuration des molécules moyennes (ou grosses pour la plasmaphérèse) sans augmenter la quantité de liquide d'échange et sans changer l'épuration standard des petites molécules (petites et moyennes pour la plasmaphérèse),
- consommer beaucoup moins de liquide stérile, donc avoir un coût moins élevé,
éliminer suffisamment de molécules de taille moyenne, garder les oligo-éléments et les nutriments qui sont réinjectés au patient, filtrer à haut volume.

Particulièrement, dans la configuration illustrée en figure 5, de nombreux autres avantages sont présents. Un nombre minimal de moyens de réglage de débit est nécessité : une pompe péristaltique 101 sur la ligne artérielle et une pompe 103 sur la canalisation 40 permettent de faire fonctionner le dispositif.

Aussi, la position des moyens de réglage de débit est intelligemment étudiée : une pompe sur la canalisation 40, et le moyen de réglage de débit 103 n'a pas besoin d'tre très puissant. Ceci permet d'avoir un fonctionnement en longue durée pour des soins intensifs en évitant une forte obturation des pores des différentes membranes.

Le fonctionnement plasmaphérèse atteint son optimum lorsque les membranes sont choisies et employées avec soin.

## Revendications

1. Dispositif de traitement extracorporel de sang comprenant:
au moins un échangeur (1) comportant au moins une première entrée (2) pour le sang à traiter, une première sortie (4) de fluide et une deuxième sortie (5) de fluide, l'échangeur (1) est un plasma-filtre, ou le plasma-filtre a un point de coupure compris entre un million et cinq million daltons,
une ligne d'entrée (10) du sang à traiter raccordée à la première entrée (2) de l'échangeur (1),
une ligne de sortie (11) du sang raccordée à la première sortie (4) de l'échangeur (1),
au moins une unité de traitement (21) comportant au moins une première entrée (22) de fluide et au moins une première sortie (24) de fluide, où l'unité de traitement (21) comprend une cartouche d'adsorption ou un réacteur capable de fixer au moins une substance donnée,
une première conduite (12) reliant la deuxième sortie (5) de l'échangeur (1) avec la première entrée (22) de l'unité de traitement (21),
une canalisation (40) reliant la première sortie (24) de l'unité de traitement 21 avec un point de branchement de la ligne d'entrée (10), pour mettre la première sortie (24) de l'unité de traitement (21) en communication de fluide avec la ligne d'entrée (10),
des moyens de réglage de débit (102) de liquide sur la deuxième canalisation (40),
**caractérisé en ce que**
le dispositif comprend des moyens de réglage de débit (103) de liquide sur la première conduite (12).

2. Dispositif selon la revendication 1 **caractérisé en ce que** l'échangeur (1) ou l'unité de traitement (21) comporte une membrane à fibres creuses ou une membrane à fibres planes.

3. Dispositif selon une quelconque des revendications précédentes comportant des moyens de réaction sur au moins certaines molécules (70) et en ce que ces moyens (70) sont branchés sur la conduite (12) entre la deuxième sortie (5) de l'échangeur (1) et la première entrée (22) de l'unité de traitement (21).

4. Dispositif selon la revendication 3 **caractérisé en ce que** les moyens de réaction sur au moins certaines molécules (70) comprennent un réacteur, ou un adsorbeur, ou un dispositif de rayonnement.

## Patentansprüche

1. Extrakorporale Blutbehandlungsvorrichtung umfassend:
mindestens einen Austauscher (1) aufweisend mindestens einen ersten Eingang (2) für das zu behandelnde Blut, einen ersten Fluidausgang (4) und einen zweiten Fluidausgang (5), wobei der Austauscher (1) ein Plasmafilter ist, wobei der Plasmafilter einen Cut-off zwischen ein Million und fünf Millionen Dalton besitzt, eine Eingangsleitung (10) für das zu behandelnde Blut, die an den ersten Eingang (2) des Austauschers (1) angeschlossen ist,
eine Blutausgangsleitung (11), die an den ersten Ausgang (4) des Austauschers (1) angeschlossen ist,
mindestens eine Behandlungseinheit (21) aufweisend mindestens einen ersten Fluideingang (22) und mindestens einem ersten Fluidausgang (24), wobei die Behandlungseinheit (21) eine Adsorptionskartusche oder einen Reaktor umfasst, die/der mindestens einen gegebenen Stoff fixieren kann,
eine erste Leitung (12), die den zweiten Ausgang (5) des Austauschers (1) mit dem ersten Eingang (22) der Behandlungseinheit (21) verbindet,
einen Kanal (40), der den ersten Ausgang (24) der Behandlungseinheit (21) mit einer Verzweigungsstelle der Eingangsleitung (10) verbindet, um den ersten Ausgang (24) der Behandlungseinheit (21) in Fluidkommunikation mit der Eingangsleitung (10) zu setzen,
Flüssigkeitsflussrateneinstellmittel (102) am zweiten Kanal (40),
**dadurch gekennzeichnet, dass**
die Vorrichtung die Flüssigkeitsflussrateneinstellmittel (103) an der ersten Leitung (12) umfasst.

2. Vorrichtung nach Anspruch 1, **dadurch gekennzeichnet, dass** der Austauscher (1) oder die Behandlungseinheit (21) eine Hohlfasermembran oder eine Flachfasermembran aufweist.

3. Vorrichtung nach einem der vorhergehenden Ansprüche, aufweisend Reaktionsmittel an mindestens einigen Molekülen (70), und dass diese Mittel (70) an der Leitung (12) zwischen dem zweiten Ausgang (5) des Austauschers (1) und dem ersten Eingang (22) der Behandlungseinheit (21) angeschlossen sind.

4. Vorrichtung nach Anspruch 3, **dadurch gekennzeichnet, dass** die Reaktionsmittel an mindestens einigen Molekülen (70) einen Reaktor oder einen Adsorber oder eine Strahlungsvorrichtung umfassen.

## Claims

1. A device for extracorporeal blood treatment comprising:
at least one exchanger (1) exhibiting at least a first inlet (2) for blood to be treated, a first fluid outlet (4) and a second fluid outlet (5), the exchanger (1) being a plasma filter, wherein the plasma filter has a cut-off point between one million and five million Daltons,
an inlet line (10) for blood to be treated, connected to the first inlet (2) of the exchanger (1), a blood outlet line (11) connected to the first outlet (4) of the exchanger (1),
at least one treatment unit (21) exhibiting at least a first fluid inlet (22) and at least a first fluid outlet (24), wherein the treatment unit (21) comprises an adsorption cartridge or a reactor capable of fixing at least one given substance,
a first tube (12) connecting the second outlet (5) of the exchanger (1) to the first inlet (22) of the treatment unit (21),
a channel (40) connecting the first outlet (24) of the treatment unit (21) to a branching point of the inlet line (10), so as to place the first outlet (24) of the treatment unit (21) in fluid communication with the inlet line (10),
liquid flow rate regulating means (102) on the second channel (40),
**characterized in that**
the device comprises liquid flow rate regulating means (103) on the first tube (12).

2. The device according to claim 1, **characterized in that** the exchanger (1) or the treatment unit (21) exhibits a hollow fiber membrane or a flat fiber membrane.

3. The device according to any one of the preceding claims, exhibiting means for reacting on at least certain molecules (70), and these means (70) are branched on the tube (12) between the second outlet (5) of the exchanger (1) and the first inlet (22) of the treatment unit (21).

4. The device according to claim 3, **characterized in that** the reaction means on at least certain molecules (70) comprise a reactor or an adsorber or a radiation device.
